# EUROPEAN PATENT SPECIFICATION

(11) **EP 2 783 661 B1**
(45) Date of publication and mention of the grant of the patent: **11.01.2017**
(21) Application number: 13162476.9
(22) Date of filing: 05.04.2013
(51) Int. Cl.: A61F 5/01

(54) **ORTHOSIS FOR ADDRESSING FOOT DROP**
ORTHESE ZUR BEHANDLUNG VON FALLFÜSSEN
ORTHÈSE DE TRAITEMENT DU PIED TOMBANT

(30) Priority: 29.03.2013 US 201313853075
(43) Date of publication of application: 01.10.2014
(73) Proprietor: Faux, Jonathan Robert, Provo, UT 84604 (US)
(72) Inventor: Faux, Jonathan Robert, Provo, UT 84604 (US)
(74) Representative: Banse & Steglich

(56) References cited:
- WO-A1-2011/126415
- GB-A- 2 478 271
- US-A1- 2008 171 956
- US-A1- 2012 029 404
- US-B2- 8 382 694

## Description

### TECHNICAL FIELD

This disclosure relates generally to orthoses and, more specifically, to orthoses that are configured of use in treating conditions that relate to feet, including foot drop.

### BACKGROUND OF RELATED ART

Foot drop, which is also known as "drop foot," is a condition in which the front part of an individual's foot, or "forefoot," and his or her toes drop. Various causes of foot drop include weakness of the forefoot, injury to the peroneal nerve, paralysis of muscles in the anterior, or front, portion of the lower leg (*i.e.,* the shin), or a variety of other conditions, such as a stroke, multiple sclerosis (MS), Charcot-Marie-Tooth disease, or others. The condition may be temporary or permanent, and can affect one foot or both of an individual's feet.

Because foot drop affects an individual's ability to lift his or her forefoot, it can cause difficulty walking. A person with foot drop may drag his or her toes along the ground when walking or lift his or her knee higher than normal while walking in order to prevent the foot from dragging, resulting in what is commonly referred to as "steppage gait." These complications may make decrease the mobility of affected individuals.

While various orthoses are available for treating foot drop, they are often difficult and clumsy to set up and adjust. Many require specialized or modified shoes. The complexity, weight, and lack of flexibility associated with many orthoses make them less convenient for affected individuals.

GB 2478271 A discloses a dorsiflexion recreation device comprising a harness, a retraction mechanism, a manchette, at least one pressure sensor and a control module.

The preamble of appended claim 1 is based on that disclosure.

US 2012/0029404 A1 discloses an ankle brace, comprising a boot assembly comprising a semi-rigid or rigid support, an adjustable stirrup, a first lace and a first rotatable tightening mechanism.

US 8,382,694 B2 discloses an ankle-foot orthotic for treating foot-drop, comprising an ankle brace, a primary strap loop and an elastic strap operably linking the ankle brace to a wearer's shoe.

WO 2011/126415 A1 discloses a device for providing support of a foot, comprising a rail, at least one strapping means, at least one guiding means and a strap.

### SUMMARY

In accordance with the present invention there is provided an orthosis as defined in independent claim 1. Preferred embodiments are defined in the dependent claims.

This disclosure, in one aspect, relates to orthoses for addressing foot drop or other conditions that may be addressed by adjusting an angle of the foot relative to the lower leg. For the sake of simplicity, such an orthosis may also be referred to herein as a "drop foot brace." A drop foot brace may be configured to exert an upward force on a foot-receiving member (*e.g*., an item of footwear, another foot-receiving member configured to be worn on an individual's forefoot, etc.) and, thus, on the individual's foot.

Various embodiments of a drop foot brace may include a support that is configured to secure the drop foot brace to a lower leg of an individual, as well as a tensioning component, which is configured to exert an upward force on a foot-receiving member and, thus, on a foot, of an individual (who may also be referred to herein as a "user").

The support of a drop foot brace includes an anterior element and an elongated securing element. The anterior element is configured to be positioned over a shin of the user's lower leg. The elongated securing element (or, more simply, an "elongated element") is configured to wrap at least partially around the user's lower leg and to secure the anterior element in place over the user's shin. In some embodiments, the support may be configured to wrap completely around the user's leg.

The tensioning component of a drop foot brace is configured to removably engage a foot-receiving member, which, in turn, may be secured to a user's foot. The tensioning component may include at least one flexible elongated element (or, more simply, an "elongated element") (*e.g*., a cord, a cable, etc.), at least two engagement elements that are configured to engage corresponding features of the foot-receiving member (*e.g*., eyelets, such as reinforced or unreinforced holes, loops or hooks, etc.) and that are actuated by the flexible elongated element, and a tensioning system configured to introduce tension in the flexible elongated element (and, optionally, to release tension from the flexible elongated element). The tensioning system comprises a reel tensioning system, in which a handle and reel may be rotated to take up the flexible elongated element, and to introduce tension therein. In addition, the tensioning component includes at least two guides for the flexible elongated element. The guides are carried by the support of the drop foot brace.

An engagement element of a tensioning component of a drop foot brace may include an upper portion, a central portion extending from the upper portion, and a lower portion adjacent to an opposite side (or end) of the central portion from the upper portion. The upper portion is configured to receive and to be engaged by the flexible elongated element, which is the elongated cinching member. In some embodiments, an aperture through the upper portion receives the flexible elongated element. The aperture may be configured to minimize wear on the flexible elongated element as the engagement element moves along the flexible elongated element or as the flexible elongated element moves through the aperture. Wear of the flexible elongated element may be
minimized by avoiding corners at locations of the upper portion that may come into contact with the flexible elongated element during use of the foot brace. As a non-limiting example, interior peripheral surfaces that define the aperture through the upper portion may be convex. The lower portion of the engagement element, which extends transverse to a length of the engagement element, may include a tooth at or near an end of the lower portion. The tooth may be configured to engage an eyelet of a foot-receiving member or the foot-receiving member without damaging the same.

The anterior element of the support includes a base and at least two collapsible regions adjacent to the base. The guides of the tensioning components are secured to the at least two collapsible regions. When tension is applied to the flexible elongated element (e.g., by the tensioning system or otherwise), the flexible elongated element may pull on the guide, which, in turn, may cause the collapsible region of the anterior element of the support to bend or collapse. In contrast, the base of the anterior element of the support has sufficient rigidity to resist collapsing under such force. Such a selectively collapsible configuration may enable the anterior element to remain in place while the tensioning component of a foot brace pulls a foot-receiving member upward.

An exemplary method for addressing drop foot includes securing a foot-receiving member to a foot of an individual; securing a support of an orthosis for addressing drop foot according to the present invention to a lower leg, above the foot; inserting engagement elements into corresponding features of the foot-receiving member; and introducing tension in a flexible elongated element of the orthosis. As tension is introduced into the flexible elongated element, the orthosis may pull the foot-receiving member and the foot upward. As tension is introduced in the flexible elongated element, a collapsible region of an anterior element of a support of the brace may collapse or bend under the force generated by the collapsible region, while the base of the support may have sufficient rigidity to resist collapsing under such force. In addition, a tooth on the end of each engagement member may engage their corresponding features (*e.g*., eyelets, etc.) of the foot-receiving member or portions of the foot-receiving member that are located immediately adjacent to those corresponding features. The orthosis may be removed by releasing tension in the flexible elongated element; removing each engagement member from its corresponding feature of the foot-receiving
member (and, optionally, disengaging a tooth of each engagement member); and removing the orthosis from the lower leg.

Other aspects, as well as the features and advantages of various aspects, of the disclosed subject matter will become apparent to those of ordinary skill in the art from the ensuing description, the accompanying figures, and the appended claims.

### BRIEF DESCRIPTION OF THE DRAWINGS

In the Figures:
FIG. 1 is a front view of an exterior surface of an embodiment of an orthosis for addressing foot drop, or "drop foot brace";
FIG. 1A illustrates an inventive embodiment of an orthosis that may be used to address drop foot, among other purposes;
FIGs. 2A through 2C are views of an embodiment of an engagement element that may be used with a drop foot brace;
FIGs. 2D through 2F illustrate another embodiment of engagement element that may be used with a drop foot brace;
FIG. 3 illustrates use of the embodiment of drop foot brace shown in FIG. 1 in conjunction with a foot-receiving member that is configured to be worn without a shoe;
FIG. 4 shows use of the embodiment of drop foot brace depicted by FIG. 1 in conjunction with a shoe with laces; and
FIG. 5 is a flow chart illustrating an embodiment of a method for using an embodiment of a drop foot brace in conjunction with a foot receiving member or a shoe.

### DETAILED DESCRIPTION

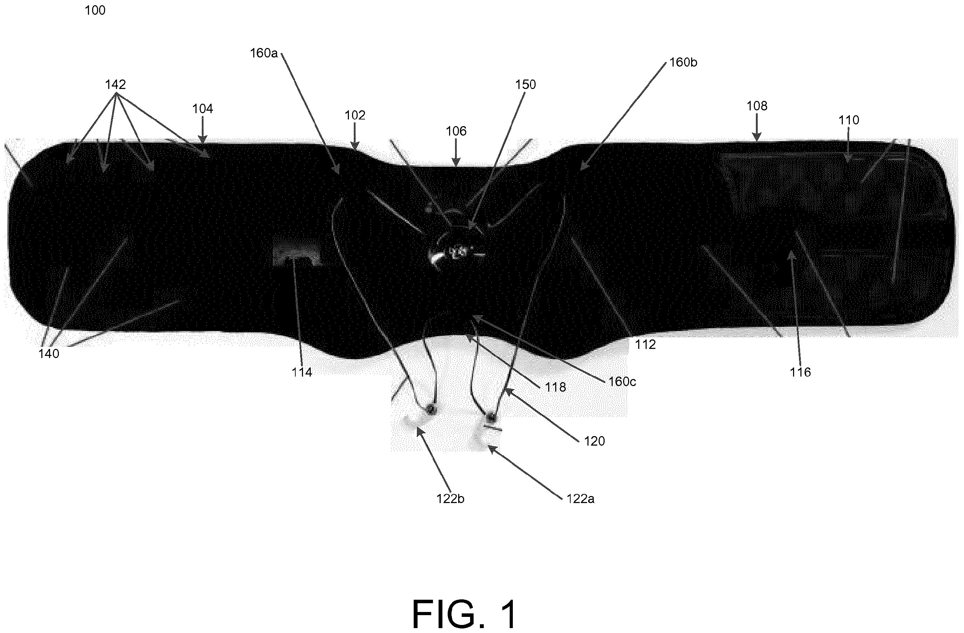

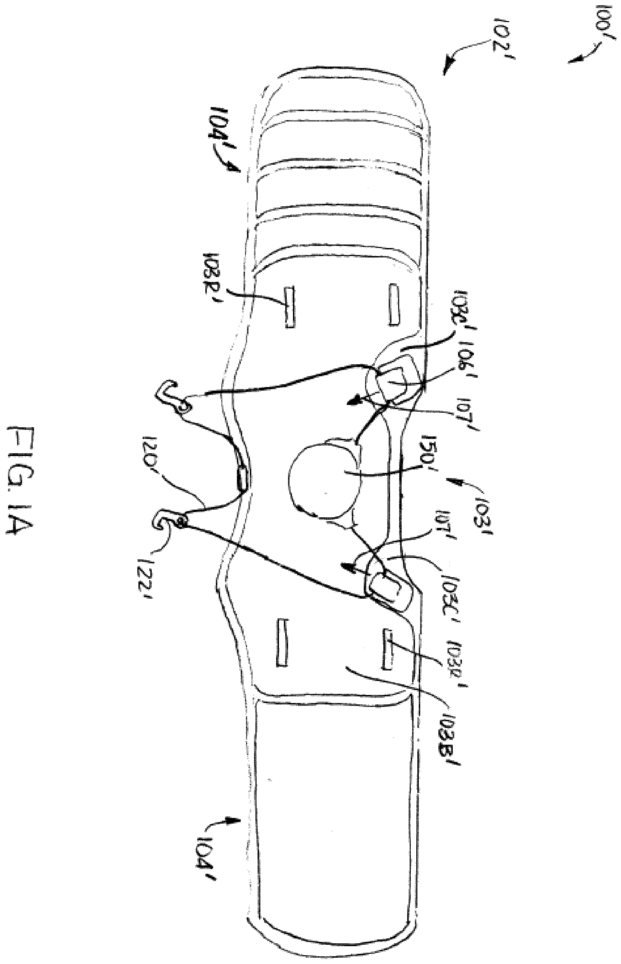

FIG. 1 illustrates one embodiment of a drop foot brace 100. The drop foot brace 100 provides support for the forefoot of the user, and can be used to treat foot drop and other conditions where the forefoot needs additional support.

The drop foot brace 100 includes a support 102 for securing the drop foot brace 100 to the leg of a user wearing the drop foot brace 100. Because the support 102 is configured to be secured to the leg of a user, its construction and the materials from which it is made may be configured to enable the support 102 to engage a user's leg in a desired manner. Thus, the support 102 may include flexible materials (*e.g.,* fabrics, polymeric films, cushioning materials, etc.), rigid or somewhat rigid materials (*e.g*., plastic or metallic structural elements, form-fitting pieces, etc.) or combinations of flexible and rigid or somewhat rigid materials.

The support 102 includes an interior surface that faces inwardly toward the leg of the user when the support 102 is being worn, and an exterior surface that faces outwardly from the leg. In some embodiments, the support 102 may be configured to wrap entirely around the leg. In other embodiments, the support 102 may be configured to physically contact only portions of the leg, such as the front of the leg and the back of the leg.

The embodiment of support 102 shown in FIG. 1 is a cuff. Other types and embodiments of supports 102 are also within the scope of the disclosed subject matter. In the embodiment depicted by FIG. 1, the support 102 comprises a band-like structure for surrounding a portion of the leg of the user of the drop foot brace 100. The support 102 may be made from a flexible material such as neoprene. The support 102 may also include stiff or rigid components. The support 102 may also include foam material or other padding to make the support 102 more comfortable when worn by the user. The support 102 may be configured to be worn against the skin of the leg and/or over an item clothing (*e.g*., hosiery, a sock, a pants leg, etc.).

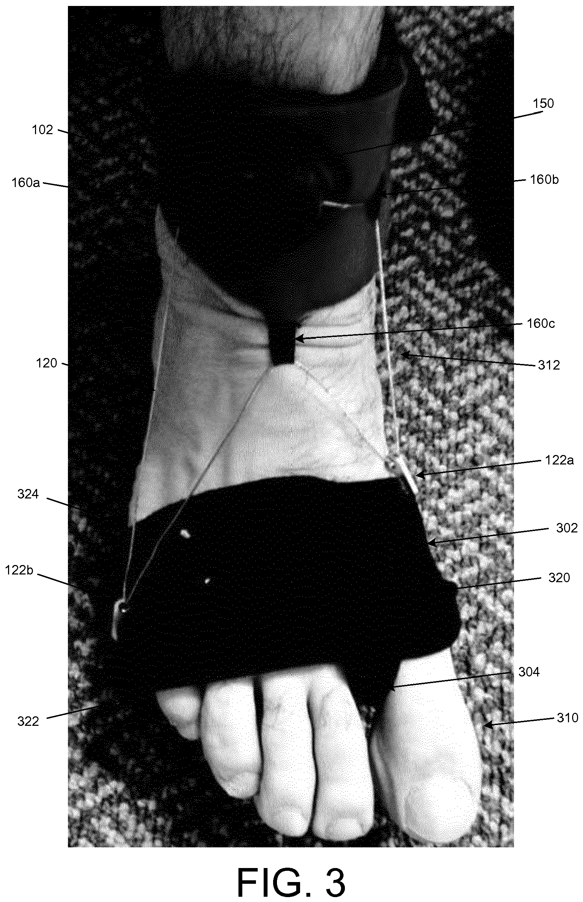

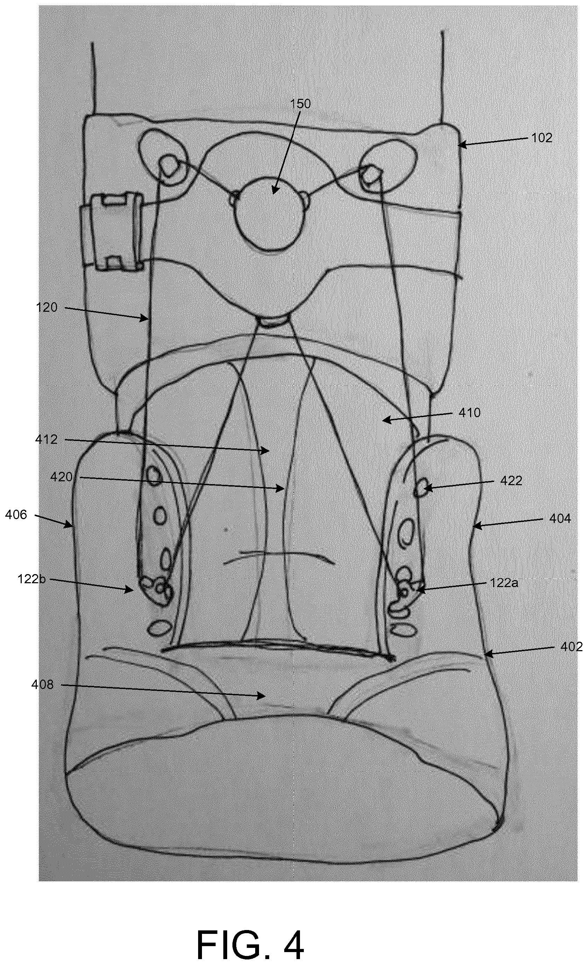

The support 102 may include a center portion 106 that is configured to be positioned on the front of the leg when being worn (as shown in connection with FIG. 3 and FIG. 4), a lateral side 104, and a medial side 108 opposite from the lateral side 104. The support 102 may include fastener (*e.g*., a hook and loop fastener (such as VELCRO^{®}), etc.) to secure the support 102 to itself and to the leg of the user. In one embodiment, the exterior surface of the medial side 108 carries a hook material 110, as shown in FIG. 1. An interior surface of the lateral side 104 may carry loop material (not seen in FIG 1). The user, when securing the support 102 in place, may wrap the medial side 108 around the leg, and then wrap the lateral side 104 around the leg such that the loop material at least partially overlaps the hook material 110.

While FIG. 1 illustrates a support 102 using hook and loop fasteners, other types of fasteners may be used instead of or in addition to hook and loop fasteners to secure the support 102 to a user's leg. The embodiment of support 102 shown in FIG. 1 also includes an optional strap 112 that at least partially secures the support 102 to the leg of the user. The strap 112 may be adjustable. It may include a securing mechanism comprising a male clip component 116 and a female clip component 114. The strap 112 may employ other forms of securing mechanisms, such as snaps, hook and loop fasteners, and others.

In one embodiment, the strap 112 (or at least a central portion thereof) may comprise a chlorosulfonated polyethylene (CSPE) synthetic rubber material (CSM), such as HYPALON^{®}. However, other appropriate materials may also be used. Such a strap 112 may also include lateral portions that comprise nylon webbing that is sewn to the central portion of the strap 112.

In use, a user may position the support 102 around his or her leg, then secure the support 102 in place with the hook material 110 and the loop material. The strap 112 may then be adjusted to further tighten or secure the support 102 to the leg. The strap 112 may be pulled around the support 102 such that the strap 112 is disposed over (and around) a majority or all of the support 102, and encircles the user's leg. The user may then adjust the tightness of the strap 112 to further secure the support 102 in position.

While FIG. 1 shows an embodiment of a support 102 with a lateral side 104 and a medial side 108 that are not connected to each othe^{r}, in other embodiments, the lateral side 104 and the medial side 108 join one another at a back of the support 102, such that the support 102 forms a unitary band. In embodiments where the support 102 comprises a unitary band, elasticity in at least a portion of the unitary band may enable the support 102 to engage and remain in place on the leg of a user. For example, the support 102 may comprise a unitary band formed from a flexible neoprene material that the user slides over his or her foot and onto his or her leg.

The support 102 is configured to fit the lower leg of the user above the ankle and below the knee, a location that is often referred to as the "crus" or the "gaiter." The center portion 106 of the support 102 may be situated proximally above the foot when being worn, as shown in FIG. 3 and FIG. 4. In certain embodiments, the center portion 106 is configured to fit an individual's leg directly above the ankle. In such an embodiment, a bottom edge of the center portion 106 may include a recess 118 for accommodating the top of an individual's foot when the support 102 is secured to the individual's lower leg. The recess 118, which comprise a concave curvature, may make the support 102 more comfortable for an individual to wear. In some embodiments, a recess 118 may enable use of the drop foot brace 100 with footwear (*e.g*., shoes, boots, etc.).

In some embodiments, a support 102 may include a number of perforations 140 that extend therethrough (*e.g.*, completely through the support 102, from the interior surface to the exterior surface; partially through the support; etc.). The perforations 140 may impart the support 102 with breathability, which may make it more comfortable to wear (*e.g*., by minimizing perspiration that might otherwise be caused by the support 102, etc.). In addition or as an alternative to perforations 140, a support 102 may include apertures (*e.g*., openings in the lateral sides 104 and 108 of the support 102, etc.). A wicking material on the interior surface of the support 102 may draw moisture away from the skin of in individual wearing the drop foot brace 100.

The support 102 may include one or more cut lines 142. Each cut line 142 may be configured to enable an individual to adjust the length of the first lateral side 104 of the support 102 by providing a location at which the first lateral side 104 may be cut to remove unneeded, excess portions of the first lateral side 104 and, thus, to ensure that the support 102 is sized appropriately for the individual who will be wearing it. In various embodiments, each cut line 142 may be configured in a manner that will help prevent fraying or other undesirable wear of the first lateral side 104 of the support 102 as it is cut (*e.g*., each cut line 142 may comprise an elongated location that is molded or otherwise compressed, etc.)to an appropriate length.

The drop foot brace 100 also includes a tensioning component that removably couples to a foot-receiving member (*e.g*., footwear, such as a shoe, a boot, etc.; etc.) worn by the user that is using the drop foot brace 100. The tensioning component couples the support 102 of the drop foot brace 100 to the foot-receiving member. The term "couple," as used herein, may refer to direct coupling with no intermediate components or indirect coupling. The tensioning component may directly couple to the foot-receiving member, or may indirectly couple to the foot-receiving member. In the embodiment shown in FIG. 1, the tensioning component is an elongated element 120 having two engagement elements 122a and 122b attached thereto.

The elongated element 120 and the two engagement elements 122a and 122b removably couple the support 102 to the foot-receiving member worn by the user. As used herein, the term "elongated element" refers to any of a variety of thin, collapsible regions along which tensile forces may be applied and maintained to provide a desired amount of bracing force *(e.g.,* a cord, a cable, a rope, a string, etc.). The elongated element 120 may comprise a single stranded element or it may include a plurality of filaments or strands that are associated with one another in a manner that imparts the elongated element 120 with strength and/or durability (*e.g*., it may comprise a plurality of twisted and/or woven strands, etc.). Any suitable material may be used to form the elongated element 120 (*e.g.,* metal wire, polyethylene, an aramid, an acrylic, or any other suitable material). In embodiments where the elongated element 120 includes a plurality of filaments or strands, ends of the filaments or strands may be secured together in a manner that prevents fraying and, thus, dissociation of the filaments or strands from one another.

The drop foot brace 100 also includes a tensioning system 150 disposed on the exterior surface of the support 102. In the illustrated embodiment, the tensioning system 150 includes a reel and a control, or handle, for winding the elongated element 120 about the reel and, thus, to adjust tension in the elongated element 120. Where the engagement elements 122b and 122a are engage features of a foot-receiving member that are configured to receive the engagement elements 122a and 122b (*e.g*., eyelets of footwear, etc.), the tensioning system 150 may pull the top of the foot-receiving member and, thus, of a foot held by the foot-receiving member, towards the support 102. In this manner, the drop foot brace 100 can provide support for an individual's forefoot and prevent the foot from dropping when the wearer lifts his or her foot while walking. In a specific embodiment, the tensioning system 150 may comprise a tensioning element of the type made by BOA Technology, Inc.

In use, the user may turn a control of the tensioning system 150 that causes the tensioning system 150 to wind the elongated element 120 about the reel. This action removes slack from the elongated element 120, and introduces tension into the elongated element 120. The tensioning system 150 may include a uni-directional control that prevents unwinding of (and a reduction of tension in) the elongated element 120 unless the user actuates a separate control to enable unwinding of the elongated element 120. Thus, the user can control the tensioning system 150 to tailor the amount of tension in the elongated element 120 and, thus, to provide his or her foot with a desired amount of support. When the user wants to remove the drop foot brace 100 or otherwise release tension in the elongated element 120, the user may use the tensioning system 150 in a manner that causes the elongated element 120 to unwind or that otherwise releases the elongated element 120.

Use of a tensioning system 150 that winds and unwinds to control the amount of tension in the elongated element 120 may be easy for an individual to use since it requires less dexterity than many other tensioning systems. Such a tensioning system 150 may be particularly useful where it is used by an individual who has suffered a stroke or other condition that affects his or her dexterity.

The elongated element 120 may have first and second ends that removably connect to the reel of the tensioning system 150, enabling the elongated element 120 to be removed from the drop foot brace 100 without removing the tensioning system 150. Removability of the elongated element 120 may be desirable for circumstances when replacement of the elongated element 120 is desired, such as when the elongated element 120 is worn or damaged.

FIG. 1 further illustrates guides that are used to position and orient the elongated element 120 relative to the drop foot brace 100 and the foot-receiving member. As used herein, the term "guide" refers to a structure that helps guide and/or position the elongated element 120. FIG. 1 illustrates an embodiment of drop foot brace 100 that includes three guides, a lateral guide 160a, a medial guide 160b, and a center guide 160c disposed on the center section 106 of the support 102.

The center guide 160c is disposed on the center portion 106 of the support 102, at or adjacent to its lower edge, which is positioned closest to a user's foot when the drop foot brace 100 is worn by the user. The elongated element 120 passes through the center guide 160c. In FIG. 1, one engagement element 122b is on one side of the center guide 160c, while the other engagement element 122a is on the other, opposite side of the center guide 160c. In the depicted embodiment, the center guide 160c comprises a loop of flexible material; however, other configurations of a center guide 160c may be used.

The drop foot brace 100 may also include a lateral guide 160a disposed on the center portion 106 of the support 102 adjacent to the lateral side 104 of the center portion 106. The lateral guide 160a may be situated above (at a higher elevation on the center portion 106 than) the center guide 160c and, in some embodiments, adjacent to an upper edge of the center portion 106 of the support 102. The lateral guide 160a may comprise a reinforced loop of flexible material, as shown in FIG. 1, or it may have any other appropriate configuration.

The drop foot brace 100 may include a similar medial guide 160b disposed on the center portion 106 adjacent to a medial side 108 of the center portion 106 and at an elevation above that of the center guide 160c. The medial guide 160b may be disposed on an opposite side of the center portion 160 from the lateral guide 160a.Like the lateral guide 160a, the medial guide 160b may comprise a reinforced loop of flexible material, or it may have any other suitable configuration.

As seen in FIG. 1, the elongated element 120 may extend from the tensioning system 150 through the lateral guide 160a, then through the engagement element 122b, which may be situated on the elongated element 120 between the lateral guide 160a and the center guide 160c.The elongated element 120 may then extend through the center guide 160c, into the engagement element 122a, through the medial guide 160b and back into the tensioning system 150.

The lateral guide 160a, medial guide 160b, and center guide 160c control the position of the elongated element 120 as the user uses the tensioning system 150 to adjust the tension in the elongated element 120. In the configuration shown in FIG. 1, the lateral guide 160a, medial guide 160b, and center guide 160c orient the elongated element 120 when it is under tension such that the elongated element 120, when secured to a foot-receiving member by the engagement elements 122a and 122b, lifts the forefoot of the user to make it easier for a person with weakness in the foot who is wearing the drop foot brace 100 to walk. Other configurations and/or arrangements of guides for the elongated element 120 that differ from those shown in FIG. 1 may also be used.

Turning now to FIG. 1A, an embodiment of support 102' of a drop foot brace 100' in accordance with the present invention is depicted. The support 102' includes an anterior element 103' and an elongated securing element 104'. The anterior element 103' is configured to be positioned over a shin of the user's lower leg. The elongated securing element 104' is configured to wrap at least partially around the user's lower leg and to secure the anterior element 103' in place on the user's shin. In some embodiments, the support 102' may be configured to wrap completely around the user's leg.

The drop foot brace 100' is configured to removably engage a foot-receiving member (*e.g*., the band 302 in FIG. 3, the footwear 402 in FIG. 4, etc.), which, in turn, may be secured to a user's foot. At least one flexible elongated element 120', at least two engagement elements 122' that are configured to engage corresponding features of the foot-receiving member (*e.g*., eyelets, such as reinforced or unreinforced holes, loops or hooks, etc.) and that are actuated by the flexible elongated element 120', and a tensioning system 150' configured to introduce tension in the flexible elongated element 120' (and, optionally, to release tension from the flexible elongated element 120') may be collectively configured to pull the foot-receiving element upward. In addition, one or more guides 160' may define a portion of the pathway of the flexible elongated element -120'. The guides 160' are carried by the support 102' of the drop foot brace 100'.

The anterior element 103' of the support 102' includes a base 103B' and a pair of collapsible regions 103C' along a top edge of the base 103B'. The collapsible regions 103C' are located on opposite sides of the support 102' and, thus, on opposite sides of the tensioning system 150'. As depicted, the collapsible regions 103C' may also be located somewhat above the tensioning system 150'.

A guide 106' is secured to each collapsible region 103C'. When tension is applied to the flexible elongated element 120' (*e.g*., by the tensioning system 150' or otherwise), the flexible elongated element 120' may pull on each of the guides 106', which, in turn, may cause the collapsible regions 103C' of the anterior element 103' of the support 102' to bend or collapse. In the embodiment illustrated by FIG. 1A, where the collapsible regions 103C' of the anterior element 103' of the support 102' are located at or near the top of the base 103B' of the anterior element 103' of the support 102', tension in the flexible elongated element 120' may pull the guides 106' and the collapsible regions 103C' down (*e.g*., in the directions of arrows 107', etc.).

In contrast, the base 103B' of the anterior element 103' of the support 102' is configured to resist collapsing under the force applied to the support 102' as tension is applied to the flexible elongated element 120'. Such resistance to collapsing may be imparted to the base 103B' in any of a variety of ways. As a few non-limiting examples, the material(s) from which the base 103B' is formed may be flexible enough to conform to the shape of an individual's shin, but impart the base 103B' with sufficient rigidity to resist undesired bending or collapsing; a construction of the base (*e.g*., laminated layers, etc.) may impart the base 103B' with the desired characteristics; reinforcement features 103R *(e.g.,* the depicted stitching, rigid reinforcements, etc.) may provide the base 103B' with the desired characteristics, etc. Juxtaposition of one or more collapsible regions 103C' and a more rigid base 103B' may make the anterior element 103' in a way that prevents undesirable bending or collapsing of the base 103B', and enable the anterior element 103' to remain in place while the flexible elongated element 120', under tension, pulls a foot-receiving member upward. The ability of the base 103' to resist collapsing may enable the support 102' to remain in place when the foot drop brace 100' is worn, which may increase user comfort.

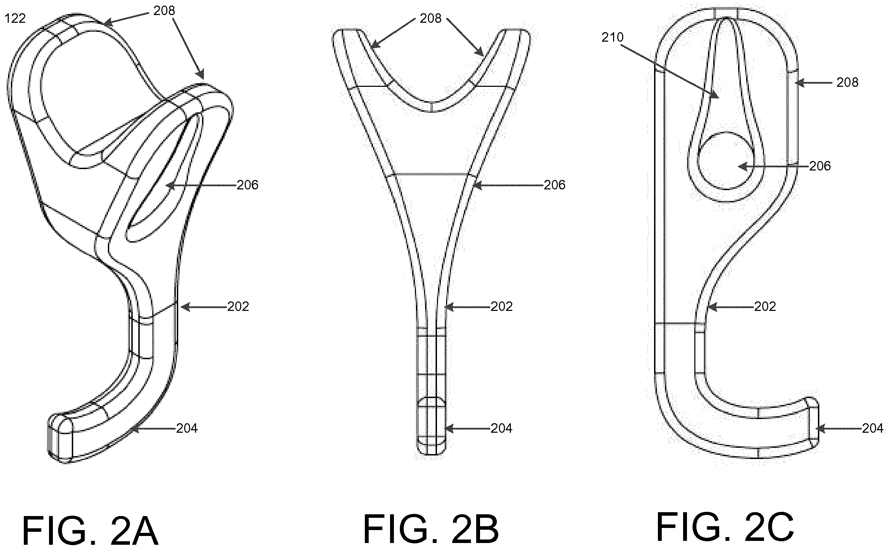

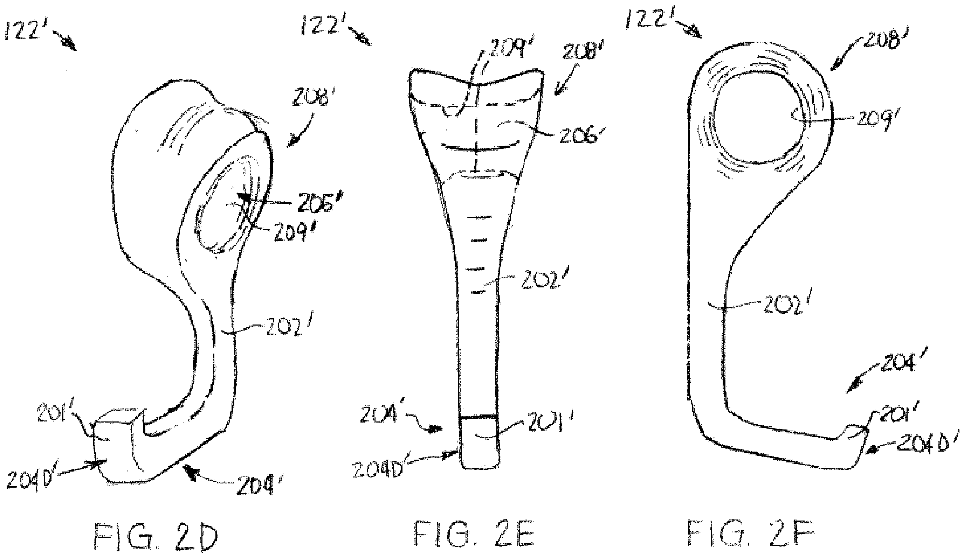

Each of FIGs. 2A-2C shows a different view of an embodiment of an engagement element 122 that may be used for the drop foot brace 100. Of course, other embodiments of an engagement element 122 that differ from the embodiment shown in FIGs. 2A-2C may also be used. FIG. 2A shows a three-quarter view of the engagement element 122. The depicted engagement element 122 includes an enlarged head 208, or upper portion, with a pair of divergently oriented flanges protruding from an upper end thereof and an aperture 206 extending laterally therethrough, from one side to the other. A vertically oriented elongated recess 210 (which, in the depicted embodiment, has a shape that resembles a teardrop) is located at each end of the aperture and extends into the side or lower surface of each flange. A neck 202, or center portion, extends from the enlarged head 208 to a curved element, or lower portion of the engagement element 122, which forms a hook 204.

The hook 204 of the engagement element 122 is configured to be coupled to a corresponding feature of the foot-receiving member (*e.g*., an eyelet, hook, loop, lace, etc., of a shoe or other footwear, etc.). Thus, when the hook 204 engages a corresponding feature of the footreceiving member, it (along with the remainder of the engagement element 122 and the elongated element 120 and other features of the tensioning component) provides the necessary connection between the support 102 and the foot-receiving member of the drop foot brace 100. The hook 204 may, as shown, terminate in a raised lip to impart it with a "J" shape. A raised lip may help reduce the likelihood that the engagement element 122 slips and accidentally uncouples from the feature of the foot-receiving member that the hook 204 engages.

In some embodiments, the support engage 122 may lack a curved hook 204, as other configurations may suitably engage corresponding features of a foot-receiving member.

The engagement element 122 may be coupled to a foot-receiving member at a location adjacent to the distal end, or front, of a user's foot. Alternatively, the engagement element 122 may engage another portion of a foot-receiving member, such as the sidewall of an item of footwear. The particular coupling options may vary based on the type of foot-receiving member and user preference.

The aperture 206 through the enlarged head 208 of the engagement element 122 may be configured to receive the elongated element 120 shown in FIG. 1 and described in reference thereto. The aperture 206 may be sized and shaped to allow the engagement element 122 to move along the length of the elongated element 120. In other embodiments, the engagement element 122 may be fixed to a certain position along the length of the elongated element 120.

The enlarged head 208 of the engagement element 122 may also define a guide, which is also referred to herein as a "hook guide" to distinguish it from the lateral guide 106a, the medial guide 106b, and the center guide 106c on the support 102, as discussed in reference to FIG. 1. In the depicted embodiment, the divergent flanges that protrude from the enlarged head 208 of the engagement element 122, the recesses 210 in the outer (or lower) surfaces of the flanges and the aperture 206 define the hook guide. The hook guide urges the elongated element 120 toward the support 102 of the drop foot brace 100 when the elongated element 120 is under sufficient tension. More specifically, the hook guide and, more specifically, the recesses 210 and the angles at which their respective flanges are oriented may control the angle at which the elongated element 120 is bent when the elongated element 120 is under sufficient tension. In some embodiments, the hook guide may provide a substantially semi-circular path for the elongated element 120. In certain embodiments, the elongated element 120 may comprise a material that may be irreversibly deformed when subjected to tensile stress that exceeds a critical threshold. The hook guide may be configured to distribute the force applied to the elongated element 120 and increase or maximize the distance over which the elongated element 120 contacts the engagement element 122. In this manner, the hook guide may prevent the elongated element 120 from exceeding its elastic and, thus, prevent or reduce permanent deformation of or other damage to the elongated element 120. Such a configuration of hook guide may also prevent bending of the elongated element 120 at sharp angles, which may reduce the possibility that the elongated element 120 will break or snap at any location where it contacts the engagement element 122.

Another embodiment of engagement element 122' is shown in FIGs. 2D-2F. The engagement element 122' includes an upper portion 208', a central portion 202' extending from the upper portion 208', and a lower portion 204' adjacent to an opposite side (or end) of the central portion 202'. The upper portion 208' is configured to receive and to be engaged by the flexible elongated element 120, 120' (FIGs. 1 and 1A). In some embodiments, an aperture 206' through the upper portion 208' receives the flexible elongated element 120, 120'. The aperture 208' may be configured to minimize wear on the flexible elongated element 120, 120' as the engagement element 122' moves along the flexible elongated element 120' or as the flexible elongated element 120' moves through the aperture 206'. Wear of the flexible elongated element 120, 120' may be minimized by providing a configuration that lacks corners at locations of the upper portion 208' that may come into contact with the flexible elongated element 120, 120' during use of the foot brace 100, 100' (FIGs. 1 and 1A). As a non-limiting example, interior peripheral surfaces 209' of the upper portion 208', which define the aperture 206', may be convex and continuous with side surfaces 208S' of the upper portion 208'.

The lower portion 204' of the engagement element 122', which extends transverse to a length of the engagement element 122', may include a tooth 201' at or near a distal end 204D' of the lower portion 204'. The tooth 201' may be configured to engage an eyelet of a foot-receiving member or the foot-receiving member when the lower portion 204' is inserted into an eyelet or other corresponding feature of a foot-receiving member and a pulling force is then applied to the engagement element 122' (*e.g*., to the upper portion 208' of the engagement element 122'. A pulling force may be applied as tension is applied to a flexible elongated element 120, 120' (FIGs. 1 and 1A) of a drop foot brace 100' (FIGs. 1 and 1A). In some embodiments, the tooth 201' of an engagement element 122' may be configured to engage its corresponding feature or a foot-receiving member without damaging the same.

FIG. 3 illustrates an embodiment of a drop foot brace 100 being worn by a user and connected to a foot-receiving member. The foot-receiving member shown in FIG. 3 comprises a band 302 that wraps around at least a portion of the foot of the user. The band 302 includes a bottom surface (not seen) for receiving the plantar portion of the foot of the user, and an upper surface 320 that is configured to be positioned over the dorsal, or top, portion of the foot of the user, as seen in FIG. 3. The band 302 also includes a proximal opening 324 for receiving the user's foot, and a distal opening 322 through which the distal end 312 *(e.g.,* toes, etc.) of the user's foot may protrude. In other embodiments, the band 302 may lack a distal opening 322 and, thus, it may be configured to cover the toes of the user.

The bottom surface of the band 302 may be made from a different material than the upper surface 320 of the band 302. For example, the bottom surface may include a durable tread to provide traction when the user is walking without any footwear or other protection on the band 302, or that helps prevent an item of footwear (*i.e.,* a sandal, etc.) worn over (or under) the band 302 from slipping off of the foot.

The band 302 may be adjustable to accommodate a variety of different sizes of feet. As an example, the upper surface 320 of the band 302 may be configured to adjust to the size of a foot. Without limitation, the upper surface 320 of such a band 302 may a length adjustable element (*e.g*., a strap, etc.). The band 302 may be configured to fit over the bare foot of the user, or over a footwear item of the user; for example, the band 302 may fit over a flip-flop, a sandal, or other item of footwear worn on the foot of the user. Such an embodiment may be desirable where the footwear does not include lace guides that can be engaged by the engagement elements 122a and 122b. In certain embodiments, the band 302 may be placed on the foot first, then the footwear item may be positioned over the band 302, as shown in FIG. 3.

Some embodiments of band 302 may include a strap 304 that removably secures the bottom surface to the upper surface 320. The strap 304 may partially close the distal opening 322 to help secure the band 302 to the foot of the user. In the embodiment shown, the strap 302 is configured and positioned to pass between the hallux (or big toe) and the second toe of the user.

The band 302 may include one or more connection points or other features to which the engagement elements 122a and 122b (or other variety of tensioning component) may connect. The connection points may be eyelets, lace guides, hooks, loops, or any other suitable connection point. The connection points may be positioned on opposite sides of the band 302.

FIG. 4 illustrates an embodiment of a drop foot brace 100 being worn by a user and connected to an item of footwear402. The footwear 402 may be a shoe, a boot, or any other type of footwear. The footwear 402 may have a high-top configuration, a mid-top configuration, or other. While the depicted embodiment shows the drop foot brace 100 as being separate from the footwear 402, in some embodiments, the drop foot brace 100 may be integral with the footwear 402. For example, the tensioning system 150 may be built into the tongue 410 or another portion of the footwear 402 (*e.g.,* in embodiments where the footwear 402 has a sufficiently high top, etc.). In such an embodiment, the support 102 may be defined by sides 404 and 406 of an upper of the footwear 402 and the tongue of the footwear 402.

In addition to sides 404 and 406, the upper of the footwear 402 may include a toe portion 408, an ankle portion 412 and a lacing area 420 between the sides 404 and 406. Lace guides 422 are positioned on opposite sides of the lacing area 420. The lace guides 422 may be eyelets, hooks, loops, or any other variety of lace guide 422. The footwear 402 includes a lace or other securing element that passes over the lacing area 420 and through the lace guides 422. The lace may be tightened to secure the footwear 402 to the foot of a user.

The user may attach the support 102 to the leg just above the footwear 402. The engagement elements 122a and 122b may be sized to removably couple to the lace guides 422 of the footwear 402. The first engagement element 122a may couple to a lace guide 422 on one side of the lacing area 420 and the second engagement element 122b may couple to a lace guide 422 on the opposite side of the lacing area 420, as shown in FIG. 4. The user may couple the engagement elements 122a and 122b to any lace guide 422 between the ankle portion 412 and the toe portion 408 of the footwear. The user may choose the lace guides 422 to which the engagement elements 122a and 122b connect based on one or more of the footwear 402, the desired level of comfort, the desired amount of support, or any other criteria. While FIG. 4 shows engagement elements 122a and 122b connecting to lace guides 422, the engagement elements 122a and 122b may connect to any suitable feature of the footwear 402.

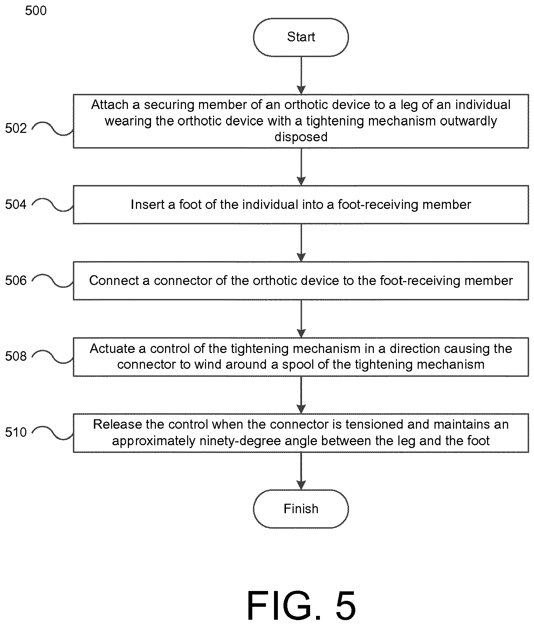

FIG. 5 shows an embodiment of a method 500 for using a drop foot brace 100 (*see* FIGs. 1, 3 and 4). The method 500 may commence at reference 502 with attaching a support of a drop foot brace 100 to a leg of an individual. The drop foot brace 100 may be attached to the leg above the ankle and below the knee such that the tensioning system 150 of the drop foot brace 100 is outwardly disposed on the surface of the support 102.

The method 500 may also involve, at reference 504, inserting the foot of the individual into a foot-receiving member. The foot-receiving member may be an item of footwear 402 (FIG. 4), a band 302 (FIG. 3), or other suitable foot-receiving member. The method 500 may further involve, at reference 506, connecting a tensioning component of the drop foot brace 100 to the foot-receiving member. The method may additionally involve connecting the tensioning component (*e.g*., the elongated element 102) to a tensioning system 150 of the drop foot brace 100, the lateral, medial, and center guides 160a-c, and the engagement elements 122a and 122b.

The method 500, at reference 508, may include tightening the elongated element 120. As a non-limiting example, a tensioning system 150 may be used in a manner that causes the tensioning component to wind around a reel of the tensioning system 150, etc.). The user may actuate the control by turning the control in a clockwise or counterclockwise direction. By winding the elongated element 120 around the reel, the user removes the slack from the connection between the tensioning system 150 and the foot-receiving member.

The method 500 may also involve, at reference 510, releasing the control when the elongated element 120 is tensioned and maintains a desired angle (*e.g.,* an approximately ninety-degree angle, etc.) between the foot and the leg. As a result, the foot of the user is provided with additional support. The user may then walk more comfortably despite issues with weakness in the foot.

The method 500 may involve other elements in addition to, or instead of, those shown in FIG. 5. For example, where the foot-receiving member is an item of footwear 402 (FIG. 4) with a plurality of lace guides and the tensioning component includes engagement elements 122a and 122b, connecting the tensioning component of the drop foot brace 100 to the foot-receiving member may involve connecting the engagement elements 122a and 122b to at least two lace guides on opposing lateral sides of the footwear 402. Where the foot-receiving member is a band 302 (FIG. 3) that is configured to wrap around at least a portion of the foot of the user and the tensioning component includes engagement elements, connecting the tensioning component may involve connecting the engagement elements to opposing lateral sides of the band 302. Where the support 102 includes a cuff, attaching the support 102 to a user's leg may involve securing the cuff around the leg of the individual at the crus. Other variations depending on the implementation are also within the scope of the present disclosure.

Although the foregoing description contains many specifics, these should not be construed as limiting the scope of any of the claims, but merely as providing illustrations of some embodiments of the disclosed subject matter. Similarly, other embodiments of the disclosed subject matter may be devised which do not depart from the scope of any of the claims. Features from different embodiments may be employed in combination. The scope of each claim is, therefore, indicated and limited only by its plain language and the legal equivalents thereto, rather than by the foregoing description. All additions, deletions and modifications to the disclosed subject matter that fall within the meaning and scope of any of the claims are to be embraced thereby. No element recited by any of the claims is intended to be a means-plus-function limitation unless the phrase "means for" is specifically used in that claim.

## Claims

1. An orthosis (100') for addressing drop foot, comprising:
an elongated cinching member (120');
a pair of engagement elements (122') secured to the elongated cinching member (120');
a reel tensioning system (150') associated with the elongated cinching member (120') and configured to releasably cinch the elongated cinching member (120');
at least two guides (106') on opposite sides of the reel tensioning system (150'); and
a support (102') configured to be secured around a lower leg of an individual, just above an ankle below the lower leg, the support (102') including:
an anterior element (103') configured to be positioned over a shin of the lower leg; and
a securing element (104') extending from at least one side of the anterior element (103') and having a flexibility sufficient to wrap around a back side of the lower leg to secure the support (102') to the lower leg,
the orthosis (100') **characterized in that** the anterior element (103') of the support (102') includes:
at least two collapsible regions (103C') to which the at least two guides (106') are secured; and
a base (103B') to which the reel tensioning system (150') is secured, the base (103B') having a rigidity sufficient to resist collapsing of a majority of the anterior element (103') as the reel tensioning system (150') introduces tension in the elongated cinching member (120') and the tension in the elongated cinching member (120') causes the at least two guides (106') to pull on the at least two collapsible regions (103C').

2. The orthosis (100') of claim 1, wherein a rigidity of the anterior element (103') of the support (102') exceeds a rigidity of the securing element (104') of the support (102').

3. The orthosis (100') of claim 2, wherein a flexibility of the securing element (104') of the support (102') exceeds a flexibility of the anterior element (103') of the support (102').

4. The orthosis (100') of claim 1, wherein the at least two collapsible regions (103C') of the anterior element (103') of the support (102') are located along a top edge of the base (103B') of the anterior element (103') of the support.

5. The orthosis (100') of any of claims 1-4, wherein the base (103B') of the anterior element (103') of the support (102') has a rigidity sufficient to resist collapsing of a majority of the anterior element (103') as the reel tensioning system (150') introduces tension in the elongated cinching member (120') and the tension in the elongated cinching member (120') causes the at least two guides (106') to move and to bend or collapse the at least two collapsible regions (103C') of the anterior element (103').

6. The orthosis (100') of any of claims 1-4, wherein the at least two collapsible regions (103C') are configured to bend or collapse in an at least partially forward direction and in an at least partially downward direction as the reel tensioning system (150') introduces tension in the elongated cinching member (120') and the tension in the elongated cinching member (120') causes the at least two guides (106') to move.

7. The orthosis (100') of any of claims 1-6, wherein each engagement element (122') of the pair of engagement elements (122') includes:
an upper portion (208) configured to be engaged by the elongated cinching member (120') of the foot orthosis, the upper portion including interior peripheral edges defining an aperture (206) that extends through a width of the upper portion (208), the aperture (206) being configured to receive the elongated cinching member (120'), the aperture (206) being configured to minimize wear on the elongated cinching member (120') as the engagement element (122') moves along the elongated cinching member (120') or the elongated cinching member (120') moves through the aperture (206);
a central portion (202) extending from the upper portion; and
a lower portion (204) on an opposite side of the central portion (202) from the upper portion (208), the lower portion (204) configured to engage an eyelet of a foot-receiving member, the lower portion (204) extending transversely to a length of the engagement element (122') and including a tooth protruding generally toward the upper portion (208).

8. The orthosis (100') of claim 7, wherein the interior peripheral edges of the upper portion (208) of the engagement element (122') and immediately adjacent regions of sides of the upper portion (208) lack corners.

9. The orthosis (100') of claim 8, wherein the interior peripheral edges of the upper portion (208) of the engagement element (122') are convex.

10. The orthosis (100') of claim 7, wherein the tooth of the lower portion (204) of the engagement element (122') is configured to engage an eyelet of the foot-receiving member without damaging an upper of the foot-receiving member.

## Patentansprüche

1. Eine Orthese (100') zur Behandlung von Fallfuß, enthaltend:
ein längliches Zuziehelement (120');
ein an dem länglichen Zuziehelement (120') befestigtes Paar Befestigungselemente (122');
Ein Rollensystem zum Spannen (150') welches mit dem Zuziehelement (120') verbunden ist und
zur lösbaren Zuziehen des Zuziehelement (120') ausgestaltet ist;
mindestens zwei Führungen (106') an entgegengesetzten Seiten des Rollensystem zum Spannen (150'); und
eine Stütze (102'), ausgestaltet zur Befestigung um den Unterschenkel eines Individuums, gerade über einem Sprunggelenk unter dem Unterschenkel, wobei die Stütze (102') beinhaltet:
ein anteriores Element (103'), ausgestaltet um über ein Schienbein des Unterschenkels positioniert zu werden; und
ein Sicherungselement (104'), welches sich von mindestens einer Seite des anterioren Elements (103') erstreckt und eine ausreichende Flexibilität zur Umwicklung der Rückseite des Unterschenkels zur Befestigung der Stütze (102') am Unterschenkel besitzt,
wobei die Orthese (100') **dadurch gekennzeichnet ist, dass** das anteriore Element (103') der Stütze (102') enthält:
mindestens zwei kollabierbare Bereiche (103C') an welche die mindestens zwei Führungen (106') befestigt sind; und
eine Basis (103B') an welche das Rollensystem zum Spannen (150') befestigt ist, wobei die Basis (103B') eine ausreichende Festigkeit besitzt, um einem Kollabieren eines Großteils des anterioren Elements (103') zu widerstehen, wenn das Rollensystem zum Spannen (150') eine Spannung im länglichen Zuziehelement (120') erzeugt und die Spannung im länglichen Zuziehelement (120') einen Zug der beiden Führungen (106') an den mindestens zwei kollabierbaren Bereichen (103C') bewirkt.

2. Die Orthese (100') gemäß Anspruch 1, wobei die Festigkeit des anterioren Elements (103') der Stütze (102') eine Festigkeit des Sicherungselements (104') der Stütze (102') übersteigt.

3. Die Orthese (100') gemäß Anspruch 2, wobei eine Flexibilität des Sicherungselements (104') der Stütze (102') eine Flexibilität des anterioren Elements (103') der Stütze (102') übersteigt.

4. Die Orthese (100') gemäß Anspruch 1, wobei die mindestens zwei kollabierbaren Bereiche (103C') des anterioren Elements (103') der Stütze (102') entlang eines oberen Randes der Basis (103B') des anterioren Elements (103') der Stütze liegen.

5. Die Orthese (100') gemäß einem der Ansprüche 1-4, wobei die Basis (103B') des anterioren Elements (103') der Stütze (102') eine ausreichende Festigkeit besitzt, um dem Kollabieren eines Großteils des anterioren Elements (103') zu widerstehen, wenn das Rollensystem zum Spannen (150') eine Spannung im länglichen Zuziehelement (120') erzeugt und die Spannung im länglichen Zuziehelement (120') die beiden Führungen (106') bewirkt, sich zu bewegen und die mindestens zwei kollabierbaren Bereichen (103C') des anterioren Elements (103') zu biegen oder zu kollabieren.

6. Die Orthese (100') gemäß einem der Ansprüche 1-4, wobei die mindestens zwei kollabierbaren Bereichen (103C') ausgestaltet sind um zu biegen oder zu kollabieren zumindest teilweise in Vorwärtsrichtung und zumindest teilweise in Abwärtsrichtung, wenn das Rollensystem zum Spannen (150') eine Spannung im länglichen Zuziehelement (120') erzeugt und die Spannung im länglichen Zuziehelement (120') die beiden Führungen (106') bewirkt, sich zu bewegen.

7. Die Orthese (100') gemäß einem der Ansprüche 1-6, wobei jedes Befestigungselement (122') des Paares der Befestigungselemente (122') beinhaltet:
einen oberen Teil (208), welcher so ausgestaltet ist, das das längliche Zuziehelement (120') der Fußorthose darin befestigt wird, wobei der obere Teil innere periphere Ränder besitzt, welche eine Öffnung (206) definieren, die sich über die Breite des oberen Teils (208) erstreckt, wobei die Öffnung (206) zur Aufnahme des länglichen Zuziehelement (120') ausgestaltet ist, die Öffnung (206) zur Minimierung der Abnutzung auf dem länglichen Zuziehelement (120') ausgestaltet ist, wenn das Befestigungselemente (122') sich entlang des länglichen Zuziehelements (120') bewegt oder wenn sich das längliche Zuziehelement (120') durch die Öffnung (206) bewegt;
einen zentralen Teil (202) welcher sich vom oberen Teil aus erstreckt; und einen unteren Teil (204) auf einer entgegengesetzten Seite des zentralen Teils (202) des oberen Teils (208), wobei der untere Teil (204) zum Einhaken in eine Öse des Fußaufnahmeelements ausgestaltet ist, der untere Teil (204) sich transversal zu einer Länge des Befestigungselements (122') erstreckt, und einen Zahn enthält der allgemein in Richtung des oberen Teils (208) hervorsteht,

8. Die Orthese (100') gemäß Anspruch 7, wobei die inneren peripheren Ränder des oberen Teils (208) des Befestigungselements (122') und direkt angrenzende Regionen von Seiten des oberen Teils (208) keine Ecken besitzen.

9. Die Orthese (100') gemäß Anspruch 8, wobei die inneren peripheren Ränder des oberen Teils (208) des Befestigungselements (122') konvex sind.

10. Die Orthese (100') gemäß Anspruch 7, wobei der Zahn des unteren Teils (204) des Befestigungselements (122') zum Einhaken einer Öse des Fußaufnahmeelements ohne Beschädigung des oberen Teils des Fußaumahmeelements ausgestaltet ist.

## Revendications

1. Orthèse (100') de traitement du pied tombant, comprenant :
un organe d'ajustement allongé (120') ;
une paire d'éléments d'engagement (122') fixés à l'organe d'ajustement allongé (120') ;
un système de tensionnement à rouleau (150') associé à l'organe d'ajustement allongé (120') et
configuré pour ajuster de manière libérable l'organe d'ajustement allongé (120') ;
au moins deux guides (106') sur des côtés opposés du système de tensionnement à rouleau (150') ; et
un support (102') configuré pour être fixé autour de la partie inférieure d'une jambe d'une personne, juste au-dessus de la cheville en dessous de la partie inférieure de la jambe, le support (102') comportant :
un élément antérieur (103') configuré pour être positionné par-dessus un tibia de la partie inférieure de la jambe ; et
un élément de fixation (104') s'étendant depuis au moins un côté de l'élément antérieur (103') et ayant une flexibilité suffisante pour s'enrouler autour d'un côté arrière de la partie inférieure de la jambe pour fixer le support (102') à la partie inférieure de la jambe,
l'orthèse (100') étant **caractérisée en ce que** l'élément antérieur (103') du support (102') comporte :
au moins deux régions repliables (103C') auxquelles sont fixés les au moins deux guides (106') ; et
une base (103B') à laquelle est fixé le système de tensionnement à rouleau (150'), la base (103B') ayant une rigidité suffisante pour résister au repliement d'une majorité de l'élément antérieur (103') à mesure que le système de tensionnement à rouleau (150') introduit une tension dans l'organe d'ajustement allongé (120') et que la tension dans l'organe d'ajustement allongé (120') provoque une traction sur les au moins deux régions repliables (103C') par les au moins deux guides (106').

2. Orthèse (100') selon la revendication 1, dans laquelle une rigidité de l'élément antérieur (103') du support (102') est supérieure à une rigidité de l'élément de fixation (104') du support (102').

3. Orthèse (100') selon la revendication 2, dans laquelle une flexibilité de l'élément de fixation (104') du support (102') est supérieure à une flexibilité de l'élément antérieur (103') du support (102').

4. Orthèse (100') selon la revendication 1, dans laquelle les au moins deux régions repliables (103C') de l'élément antérieur (103') du support (102') sont situées le long d'un bord supérieur de la base (103B') de l'élément antérieur (103') du support.

5. Orthèse (100') selon l'une quelconque des revendications 1 à 4, dans laquelle la base (103B') de l'élément antérieur (103') du support (102') présente une rigidité suffisante pour résister au repliement d'une majorité de l'élément antérieur (103') à mesure que le système de tensionnement à rouleau (150') introduit une tension dans l'organe d'ajustement allongé (120') et que la tension dans l'organe d'ajustement allongé (120') provoque le déplacement des au moins deux guides (106') et le fléchissement ou le repliement des au moins deux régions repliables (103C') de l'élément antérieur (103') par les au moins deux guides (106').

6. Orthese (100') selon l'une quelconque des revendications 1 à 4, dans laquelle les au moins deux régions repliables (103C') sont configurées pour fléchir ou se replier dans une direction au moins en partie vers l'avant et dans une direction au moins en partie vers le bas à mesure que le système de tensionnement à rouleau (150') introduit une tension dans l'organe d'ajustement allongé (120') et que la tension dans l'organe d'ajustement allongé (120') entraîne le déplacement des au moins deux guides (106').

7. Orthèse (100') selon l'une quelconque des revendications 1 à 6, dans laquelle chaque élément d'engagement (122') de la paire d'éléments d'engagement (122') comporte :
une portion supérieure (208) configurée pour être mise en prise avec l'organe d'ajustement allongé (120') de l'orthèse du pied, la portion supérieure comportant des bords périphériques intérieurs définissant une ouverture (206) qui s'étend à travers une largeur de la portion supérieure (208), l'ouverture (206) étant configurée pour recevoir l'organe d'ajustement allongé (120'), l'ouverture (206) étant configurée pour minimiser l'usure sur l'organe d'ajustement allongé (120') à mesure que l'élément d'engagement (122') se déplace le long de l'organe d'ajustement allongé (120') ou que l'organe d'ajustement allongé (120') se déplace à travers l'ouverture (206) ;
une portion centrale (202) s'étendant depuis la portion supérieure ; et
une portion inférieure (204) sur un côté de la portion centrale (202) opposé à la portion supérieure (208), la portion inférieure (204) étant configurée de manière à venir en prise avec un sillet d'un organe de réception du pied, la portion inférieure (204) s'étendant transversalement à une longueur de l'élément d'engagement (122') et comportant une dent faisant saillie généralement vers la portion supérieure (208).

8. Orthèse (100') selon la revendication 7, dans laquelle les bords périphériques intérieurs de la portion supérieure (208) de l'élément d'engagement (122') et les régions immédiatement adjacentes des côtés de la portion supérieure (208) n'ont pas de coins.

9. Orthèse (100') selon la revendication 8, dans laquelle les bords périphériques intérieurs de la portion supérieure (208) de l'élément d'engagement (122') sont convexes.

10. Orthèse (100') selon la revendication 7, dans laquelle la dent de la portion inférieure (204) de l'élément d'engagement (122') est configurée pour venir en prise avec un oeillet de l'organe de réception du pied sans endommager une partie supérieure de l'organe de réception du pied.
